# FASCICULE DE BREVET EUROPEEN

(11) **EP 3 028 675 B1**
(45) Date de publication et mention de la délivrance du brevet: **19.07.2017**
(21) Numéro de dépôt: 14196506.1
(22) Date de dépôt: 05.12.2014
(51) Int. Cl.: A61F 2/30, A61B 90/00, A61B 34/00

(54) **Sélection graphique d'une prothèse à ancrage osseux**
Grafische Auswahl einer Prothese mit Knochenverankerung
Graphical selection of a prosthesis with bone anchor

(43) Date de publication de la demande: 08.06.2016
(73) Titulaire: X.Nov IP Sarl, 1882 Luxembourg (LU)
(72) Inventeur: Biégun, Jean-François, 2900 Porrentruy (CH)
(74) Mandataire: Lecomte & Partners

(56) Documents cités:
- WO-A1-02/071987
- WO-A1-2011/053491
- FR-A1- 2 913 592
- US-A1- 2009 274 271

## Description

### Domaine technique

L'invention a trait au domaine médical de sélection d'implant ou de prothèse, plus particulièrement d'implant ou prothèse à ancrage osseux, destiné à être posé sur un patient. Plus particulièrement, l'invention à la sélection d'un implant notamment orthopédique, par exemple de hanche ou de genou.

### Technique antérieure

Le document de brevet publié FR 2 913 592 A1 divulgue un procédé de détermination de la dimension d'une prothèse. Il consiste essentiellement à réaliser une image radiologique numérique de la zone du patient qui est destinée à recevoir la prothèse et à superposer à cette image un calque d'une prothèse d'une taille donnée afin de vérifier sa compatibilité avec la morphologie osseuse du patient. Afin de permettre une mise en correspondance des échelles respectives de l'image radiographique et du calque, le patient est équipé d'une ceinture en matériau transparent aux rayons X et supportant une bille de référence en matériau radio-opaque. La bille est disposée latéralement à la zone à radiographier, de manière à ce que sa représentation sur l'image radiologique permette d'en déduire son échelle. Le procédé de cet enseignement prévoit dans le cadre d'un traitement manuel de l'image ainsi produite, de mesurer manuellement le diamètre de l'image de la bille de référence et ensuite de choisir un jeu de calques d'une échelle appropriée. Dans le cadre d'une mise en oeuvre numérique de cet enseignement, il est prévu une analyse numérique de l'image de la bille de manière à en déterminer de manière automatique son diamètre. Un coefficient d'agrandissement ou de diminution de l'image par rapport à une échelle de référence des calques peut alors être calculé et la taille des calques est alors modifiée selon ce coefficient.

Le mode numérique de cet enseignement est intéressant en ce qu'il est essentiellement automatique. Il présente toutefois une étape potentiellement critique qui est celle de la détermination du diamètre de l'image de la bille de référence. Elle implique l'utilisation d'un filtre de lissage de l'image déterminant le gradient de la variation d'énergie des pixels entre l'image de la bille de référence et la zone environnante et ainsi déterminant le contour de la bille sur l'image. Cette opération est coûteuse en ressources informatiques et peut dans certaines situations s'avérer imprécise au point de conduire à une mise à échelle erronée. L'utilisateur n'a par ailleurs aucun moyen intuitif pour vérifier la cohérence du coefficient de correction d'échelle.

### Résumé de l'invention

### Problème technique

L'invention a pour objectif de proposer un procédé de sélection d'une prothèse destinée à être posée sur un patient, qui soit plus simple à mettre en oeuvre et plus robuste quant à la mise en correspondance des échelles respectives de l'image radiologique et des calques de prothèses.

### Solution technique

L'invention a pour objet un procédé de sélection d'une prothèse destinée à être posée sur un patient, comprenant les étapes suivantes:
(a) obtention sous forme numérique d'une image radiologique de la zone du corps du patient destinée à recevoir la prothèse, l'image comprenant la représentation d'un objet radio-opaque de référence, disposé à proximité de ladite zone ;
(b) superposition sur l'image radiologique d'un calque numérique représentant la prothèse ou un élément de ladite prothèse en vue de son positionnement ;
remarquable en ce que à l'étape (b), le calque comprend un repère destiné à être superposé à la représentation de l'objet de référence par déplacement du calque, ledit calque pouvant être agrandi ou diminué de taille de manière à ce que la taille du repère corresponde à celle de la représentation de l'objet de référence, de manière à mettre en correspondance les échelles respectives de l'image radiologique et du calque. La variation de taille du calque correspond à une transformation géométrique dite de similitude. En d'autres termes, la transformation géométrique conserve la forme et les proportions de l'image ainsi transformée.

De manière préférentielle, la taille de l'image radiologique n'est pas modifiée.

Selon un mode avantageux de l'invention, le repère est une surface ou un contour circulaire.

Selon un mode avantageux de l'invention, le repère est disposé latéralement à la représentation de la prothèse ou de son élément.

Selon un mode avantageux de l'invention, la ou les dimensions extérieures du repère sont corrélées avec les dimensions de la représentation de la prothèse ou de son élément.

Selon un mode avantageux de l'invention, le procédé est configuré pour que l'étape (b) soit réalisée par une intervention manuelle, préférentiellement via une interface homme-machine.

Selon un mode avantageux de l'invention, à l'étape (b), le déplacement du calque et sa mise à échelle avec l'image radiologique est réalisée par une intervention manuelle, préférentiellement via une interface homme-machine.

Selon un mode avantageux de l'invention, l'étape (b) comprend la sélection de la prothèse ou de son élément parmi une pluralité de prothèses ou d'éléments de prothèse, chacune des prothèses ou chacun de ses éléments étant représenté sur un calque spécifique, chacun de ces calques comprenant un repère correspondant au repère destiné à être superposé à la représentation de l'objet de référence.

Selon un mode avantageux de l'invention, la pluralité de prothèses ou d'éléments de prothèse correspond à différentes tailles d'un type donné, préférentiellement différentes tailles de tige fémorale d'une prothèse orthopédique.

Selon un mode avantageux de l'invention, la sélection à l'étape (b) est opérée via une interface graphique.

Selon un mode avantageux de l'invention, l'interface graphique comprend un listage des tailles de prothèse ou d'élément de prothèse disponibles, des moyens de commande de variation de la taille du calque sélectionné et/ou des moyens de commande de déplacement en rotation dudit calque.

Selon un mode avantageux de l'invention, le listage des tailles de prothèse ou d'élément de prothèse sur l'interface graphique comprend des surfaces spécifiques sélectionnables au moyen d'un pointeur mobile sur ladite interface et commandé manuellement.

Selon un mode avantageux de l'invention, les moyens de commande de variation de taille et/ou en rotation comprennent un curseur déplaçable par un pointeur mobile commandé manuellement.

Selon un mode avantageux de l'invention, la prothèse est constituée de plusieurs éléments destinés à être assemblés, chacun desdits éléments étant représenté sur un calque spécifique ; l'étape (b) correspondant à un premier de ces éléments, la mise en correspondance des échelles respectives de l'image radiologique et du calque du premier élément opérant de manière automatique la mise en correspondance des échelles respectives des calques des éléments restants avec l'échelle de l'image radiologique.

Selon un mode avantageux de l'invention, la prothèse est constituée de plusieurs éléments destinés à être assemblés, chacun desdits éléments étant représenté sur un calque spécifique ; l'étape (b) correspond à un premier de ces éléments et le procédé comprend l'étape supplémentaire suivante :
(c) superposition sur l'image d'un calque numérique représentant un des éléments restants de la prothèse, en vue de son positionnement, l'échelle dudit calque étant mise en correspondance avec l'échelle de l'image radiologique par l'étape (b).

Selon un mode avantageux de l'invention, l'étape (c) est itérée pour chaque élément.

Selon un mode avantageux de l'invention, au moins un des éléments de la prothèse, préférentiellement chacun de ces éléments, comprend plusieurs tailles disponibles.

Selon un mode avantageux de l'invention, la prothèse est une prothèse orthopédique de hanche comprenant une tige fémorale et un cotyle destiné à être fixé au bassin et à coopérer avec la tige.

Selon un mode avantageux de l'invention, la prothèse est une prothèse orthopédique de genou comprenant un implant tibial et un implant fémoral.

L'invention a également pour objet un dispositif configuré pour mettre en oeuvre un procédé de sélection d'une prothèse destinée à être posée sur un patient, remarquable en ce que le procédé est conforme à l'invention.

L'invention a également pour objet un programme d'ordinateur comprenant un code interprétable par un ordinateur, remarquable en ce que ledit code est configuré pour mettre en oeuvre le procédé selon l'invention.

### Avantages apportés

Les mesures de l'invention sont intéressantes en ce qu'elles permettent de réaliser une mise à l'échelle de manière simple et robuste. L'utilisateur identifie le repère du calque et sa mise en correspondance avec la représentation de l'objet radio-opaque de référence. Cette opération peut n'être réalisée qu'une seule fois. Elle évite la génération d'erreur, notamment lorsque la représentation de l'objet radio-opaque de référence est de mauvaise qualité.

### Brève description des dessins

La figure 1 est une image radiologique de la zone fémorale et du bassin d'un patient, l'image comprenant la représentation d'un objet radio-opaque de référence.
La figure 2 est l'image radiologique de la figure 1 sur laquelle est superposé un premier calque correspondant à la tige fémorale d'une prothèse de hanche, le calque comprenant un repère ;
La figure 3 correspond à la figure 2 où le premier calque a été déplacé afin de superposer son repère à la représentation d'un objet de référence.
La figure 4 correspond à la figure 3 où le calque a été diminué de taille de manière à ce que la taille de son repère corresponde à celle de la représentation d'un objet de référence.
La figure 5 correspond à la figure 4 où le premier calque a été déplacé en translation et en rotation afin de positionner la représentation de la tige sur le fémur.
La figure 6 correspond à la figure 5 où un deuxième calque correspondant à la représentation d'un cotyle destinée à être fixée dans la cavité cotyloïdienne du bassin, le calque comprenant également un repère.
La figure 7 correspond à la figure 6 où le deuxième calque a été déplacé en translation et en rotation afin de positionner la représentation du cotyle sur le bassin.
La figure 8 est une représentation d'une interface de commande graphique apte à sélectionner les calques, à modifier leur échelle et à les faire pivoter.

### Description des modes de réalisation

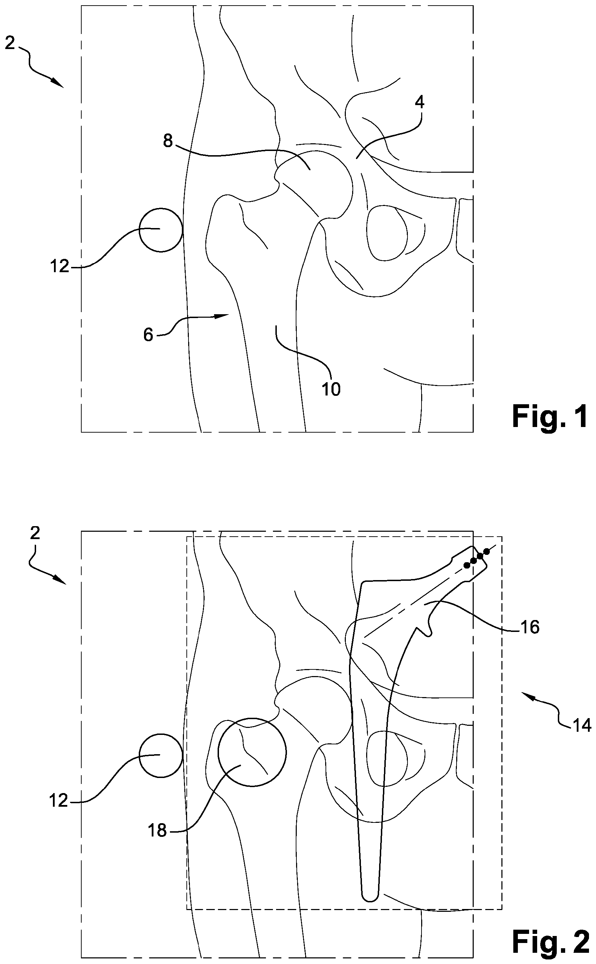

La figure 2 illustre une image radiologique 2 de la zone fémorale et du bassin d'un patient, destinée à recevoir un implant ou prothèse totale de hanche. Une prothèse totale de hanche comprend usuellement une tige en métal destinée à être scellée dans l'extrémité supérieure 10 du fémur 6, une tête prothétique supportée par la tige, et un cotyle (ou une cupule) qui est la partie supérieure de la prothèse totale de hanche, destinée à être fixée dans la cavité cotyloïdienne 8 creusée naturellement dans le bassin 4. Le cotyle est composé d'une partie métallique hémisphérique dont la surface convexe assure un contact intime avec l'os du bassin, et une garniture articulaire appelée insert qui est en céramique, en métal ou en polyéthylène et articulée avec la tête prothétique. Une prothèse totale de hanche est bien connue en soi de l'homme de métier et ne sera par conséquent pas davantage détaillée.

L'image radiologique 2 de figure 1 est prise avec des rayons X de manière bien connue en soi de l'homme de métier. Elle présente toutefois la particularité qu'un objet radio-opaque de référence, tel qu'une bille en acier, a été disposé à proximité de la zone du patient qui est destinée à recevoir la prothèse. De manière préférentielle, la bille est disposée dans la poche du pantalon du patient ou de la robe de la patiente de manière à être essentiellement à hauteur du fémur dans une direction généralement perpendiculaire au plan moyen du corps. En l'absence de poche ou lorsque l'éventuelle poche ne permet pas un positionnement adéquat, l'objet de référence peut être disposé par tout autre moyen, tel que notamment une ceinture pourvue de moyens de fixation à l'objet. L'image 2 comprend ainsi, outre une représentation de la morphologie osseuse du patient, une représentation 12 de l'objet de référence.

L'image 2 est numérisée, préférentiellement sous forme d'une image matricielle ou encore « bitmap » de manière à pouvoir être visualisée sur une interface graphique telle qu'un écran d'ordinateur.

La figure 2 illustre l'image radiologique 2 de la figure 2 sur laquelle est superposé un premier calque 14 (représenté en trait interrompu) représentant une tige fémorale 16 de prothèse de hanche, la tige présentant une taille donnée parmi un éventail de tailles différentes. En l'occurrence il s'agit de la taille 12, cette taille se rapportant essentiellement à sa longueur. Le calque 14 comprend, outre la représentation de la tige fémorale 16, une surface circulaire 18 formant un repère de l'échelle du calque. Ce repère 18 présente une taille, en l'occurrence un diamètre, qui est directement liée à l'échelle de représentation de la tige fémorale 16. Ce repère 18 est destiné à être mis en correspondance avec la représentation 12 de l'objet de référence de l'image radiologique 2.

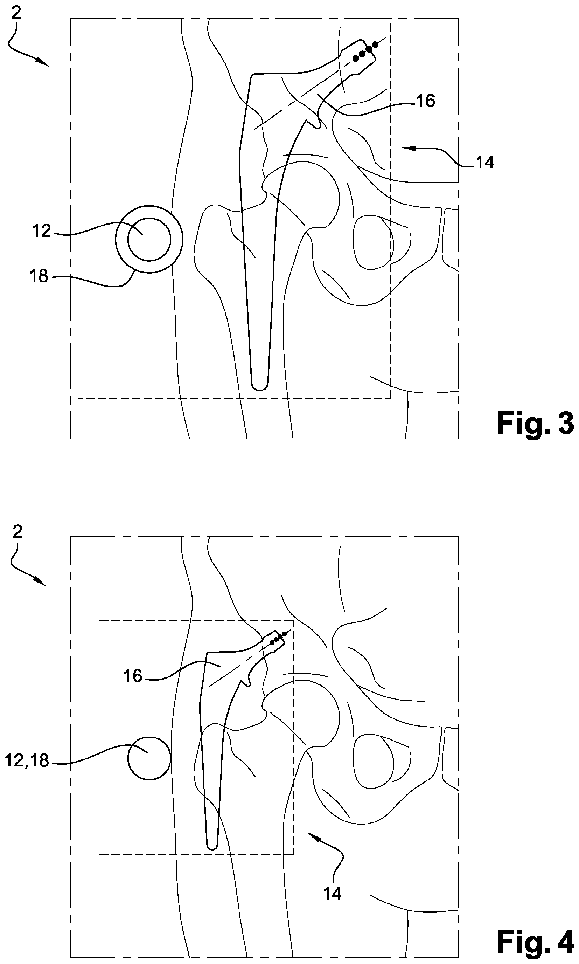

La figure 3 correspond à figure 2 où toutefois le calque 14 a été déplacé par translation de manière à amener son repère 18 en superposition sur la représentation 12 de l'objet de référence de l'image radiologique.

La figure 4 correspond à la figure 3 où la dimension du calque a été réduite de manière à ce que la dimension de son repère 18 corresponde à la dimension correspondante de la représentation 12 de l'objet de référence de l'image radiologique 2. La variation de taille du calque est une transformation géométrique du type similitude qui multiplie toutes les distances par une constante fixe, appelée son rapport, et donc conserve la forme et les proportions. Par conséquent, cette transformation modifie l'échelle du calque de manière à correspondre à celle de l'image radiologique pour autant que la dimension du repère corresponde, selon l'échelle du calque, à celle de l'objet de référence.

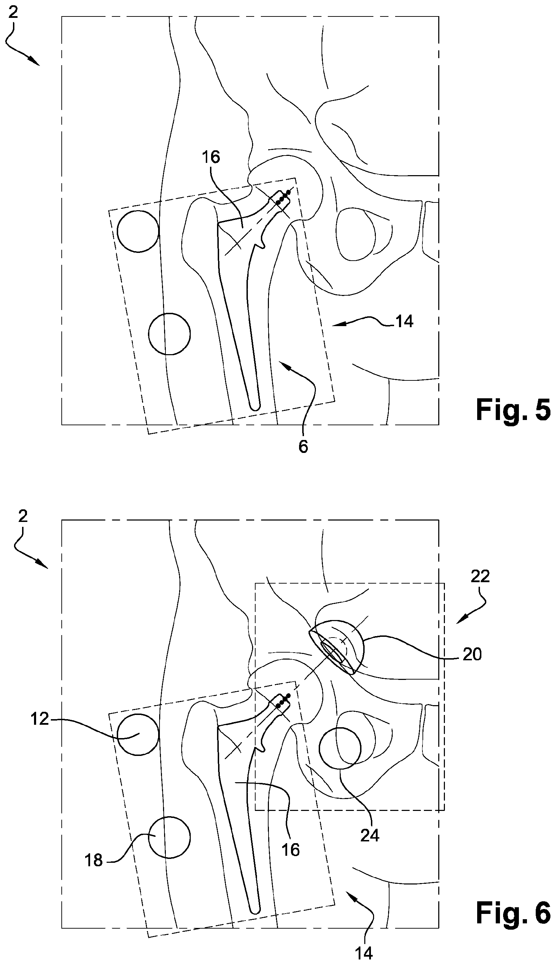

La figure 5 correspond à la figure 4 où le calque 14 a été déplacé par translation et rotation, sans toutefois modifier sa taille, de manière à amener la représentation de la tige sur la partie supérieure du fémur. Cette opération est destinée à vérifier de manière visuelle la compatibilité entre un modèle de prothèse, en l'occurrence une tige fémorale d'une taille donnée, et le ou les os avec lequel ou lesquels la prothèse est destinée à coopérer. En l'occurrence, la compatibilité semble donnée. En cas de non compatibilité, il convient alors de fermer ou faire disparaître ce calque et d'en faire apparaître une autre correspondant à un autre modèle et/ou une autre taille, et ainsi de suite jusqu'à obtenir le modèle qui convient.

La figure 6 correspond à la figure 5 où un deuxième calque 22 (également représentée en trait interrompu) est superposé à l'image radiologique 2. Ce deuxième calque comporte une représentation d'un cotyle 20 d'une taille donnée, en l'occurrence de diamètre 48mm. Ce cotyle 20 est destiné à être logé dans la cavité cotyloïdienne du bassin. Le deuxième calque 22 comprend, outre la représentation du cotyle, un repère 24 similaire ou identique au repère 12 du premier calque dont l'échelle a été mise en correspondance avec celle de l'image radiologique 2. Il est intéressant de noter que la dimension de ce repère 24 est identique à celle du premier calque mis en correspondance ainsi qu'à la représentation de l'objet de référence. En effet, selon un mode préféré de réalisation de l'invention, la mise à l'échelle du premier calque a pour effet de mettre automatiquement à l'échelle les autres calques disponibles. Cette opération est relativement simple et robuste à mettre en oeuvre dans la mesure où il suffit d'appliquer aux autres calques le même coefficient que celui résultant de la mise en correspondance manuelle du premier calque.

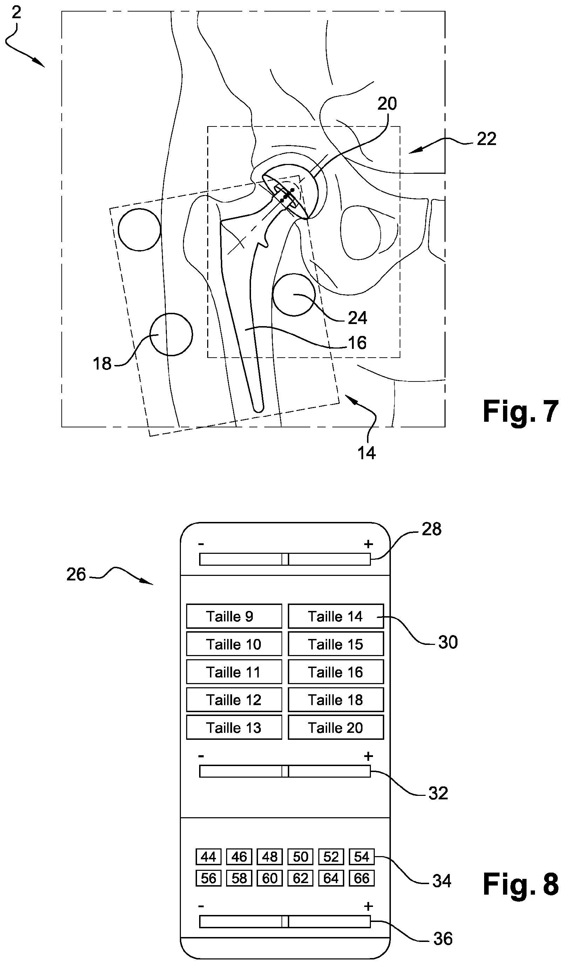

La figure 7 correspond à la figure 6 où le deuxième calque 22 comprenant le cotyle 20 a été déplacé par translation et/ou rotation afin d'amener le cotyle à l'endroit du bassin où il est destiné à être mise en place. Cette opération permet ainsi, similairement à l'opération relative au positionnement de la tige et illustrée à la figure 5, de vérifier la compatibilité géométrique du cotyle sélectionné avec la morphologie osseuse du patient, en l'occurrence celle de son bassin.

La figure 8 illustre un exemple d'interface graphique de commande pour réaliser les opérations décrites précédemment. L'interface graphique 26 peut être affichée latéralement à l'image radiologique des figures 1 à 7. Alternativement, il peut s'agir d'un menu déroulant ou encore d'une fenêtre que l'on peut ouvrir, fermer et déplacer à sa guise. Cette interface 26 comprend une série de surfaces 30 aptes à commander les calques des différentes tailles de tige, ces tailles étant repérées par les chiffres 9 à 20 apparaissant sur ces surfaces. Celles-ci peuvent être activées à la manière d'un bouton au moyen d'un pointeur mobile sur l'écran, commandé par une interface homme-machine telles qu'une souris d'ordinateur.

L'interface graphique de commande 26 comprend également un premier curseur 28 destiné à faire varier la taille du calque de tige appelé par un des boutons 30. L'interface comprend également un deuxième curseur 32 destiné à faire pivoter le calque. Le déplacement par translation du calque est préférentiellement assuré par un pointeur mobile que l'on active en enfonçant le bouton de la souris d'ordinateur lorsqu'il est sur le calque, plus particulièrement sur son repère.

L'interface graphique de commande 26 comprend également des surfaces ou boutons d'appel d'un deuxième calque correspondant aux différentes tailles de cotyle, ces tailles étant exprimées en millimètre par les chiffres allant 44 à 66 apparaissant sur ces boutons.

L'interface comprend également un troisième curseur 36 destiné à faire pivoter le calque du cotyle appelé par un des boutons 34.

Il est toutefois à noter que l'interface qui vient d'être décrite peut subir des modifications, notamment en fonction du type de prothèse à sélectionner.

L'invention qui vient d'être décrite est destinée à être mise en oeuvre via un programme informatique comprend un code apte à être interprété par un ordinateur. Ce programme peut être mis en oeuvre sur un serveur et des postes de travail dits « clients ».

## Revendications

1. Procédé de sélection d'une prothèse (16 ; 20) destinée à être posée sur un patient, comprenant les étapes suivantes :
(a) obtention sous forme numérique d'une image radiologique (2) de la zone du corps du patient destinée à recevoir la prothèse (16; 20), l'image comprenant la représentation d'un objet radio-opaque de référence (12) disposé à proximité de ladite zone ;
(b) superposition, sur l'image radiologique (2), d'un calque numérique (14) représentant la prothèse ou un élément (16 ; 20) de ladite prothèse en vue de son positionnement ;
**caractérisé en ce que**
à l'étape (b), le calque (14 ; 22) comprend un repère (18 ; 24) destiné à être superposé à la représentation de l'objet de référence (12) par déplacement du calque, la taille dudit calque étant variée de manière à ce que la taille du repère (18 ; 24) corresponde à celle de la représentation de l'objet de référence (12), de manière à mettre en correspondance les échelles respectives de l'image radiologique (2) et du calque (14 ; 22).

2. Procédé selon la revendication 1, **caractérisé en ce que** le repère est une surface ou un contour circulaire (18 ; 24).

3. Procédé selon l'une des revendications 1 et 2, **caractérisé en ce que** le repère (18 ; 24) est disposé latéralement à la représentation de la prothèse ou de son élément (16 ; 20).

4. Procédé selon l'une des revendications 1 à 3, **caractérisé en ce que** la ou les dimensions extérieures du repère (18 ; 24) sont corrélées avec les dimensions de la représentation de la prothèse ou de son élément (16 ; 20).

5. Procédé selon l'une des revendications 1 à 4, **caractérisé en ce qu'**il est configuré pour que l'étape (b) soit réalisée par une intervention manuelle, préférentiellement via une interface homme-machine, et préférentiellement à l'étape (b), le déplacement du calque (14 ; 22) et sa mise à échelle avec l'image radiologique (2) est réalisée par une intervention manuelle, préférentiellement via une interface homme-machine.

6. Procédé selon l'une des revendications 1 à 5, **caractérisé en ce que** l'étape (b) comprend la sélection de la prothèse ou de son élément (16 ; 20) parmi une pluralité, chacune des prothèses ou chacun de ses éléments étant représenté sur un calque spécifique, chacun de ces calques comprenant un repère correspondant au repère destiné à être superposé à la représentation de l'objet de référence, et préférentiellement la pluralité de prothèses ou d'éléments de prothèse correspond à différentes tailles d'un type donné, préférentiellement différentes tailles de tige fémorale (16) d'une prothèse orthopédique (16 ; 20).

7. Procédé selon la revendication 6, **caractérisé en ce que** la sélection à l'étape (b) est opérée via une interface graphique (26).

8. Procédé selon la revendication 7, **caractérisé en ce que** l'interface graphique (26) comprend un listage (30 ; 34) des tailles de prothèse ou d'élément de prothèse disponibles, des moyens de commande (28) de variation de la taille du calque (14) sélectionné et/ou des moyens de commande de déplacement en rotation (32 ; 36) dudit calque, et préférentiellement le listage des tailles prothèse ou d'élément de prothèse sur l'interface graphique (26) comprend des surfaces spécifiques (30 ; 34) sélectionnables au moyen d'un pointeur mobile sur ladite interface et commandé manuellement.

9. Procédé selon la revendication 8, **caractérisé en ce que** les moyens de commande de variation de taille et/ou en rotation (28 ; 32 ; 36) comprennent un curseur déplaçable par un pointeur mobile commandé manuellement.

10. Procédé selon l'une des revendications 1 à 9, **caractérisé en ce que** la prothèse est constituée de plusieurs éléments (16 ; 20) destinés à être assemblés, chacun desdits éléments étant représenté sur un calque spécifique (14 ; 22) ; l'étape (b) correspondant à un premier de ces éléments, la mise en correspondance des échelles respectives de l'image radiologique (2) et du calque (14) du premier élément (16) opérant de manière automatique la mise en correspondance des échelles respectives des calques (22) des éléments restants (20) avec l'échelle de l'image radiologique (2).

11. Procédé selon l'une des revendications 1 à 10, **caractérisé en ce que** la prothèse est constituée de plusieurs éléments (16 ; 20) destinés à être assemblés, chacun desdits éléments étant représenté sur un calque spécifique (14 ; 22) ; l'étape (b) correspondant à un premier (16) de ces éléments, le procédé comprend l'étape supplémentaire suivante :
(c) superposition sur l'image radiologique (2) d'un calque numérique (22) représentant un (20) des éléments restants de la prothèse, en vue de son positionnement, l'échelle dudit calque (22) étant mise en correspondance avec l'échelle de l'image radiologique (2) par l'étape (b), et
préférentiellement l'étape (c) est itérée pour chaque élément.

12. Procédé selon la revendication 11, **caractérisé en ce qu'**au moins un des éléments de la prothèse (16 ; 20), préférentiellement chacun de ces éléments, comprend plusieurs tailles disponibles.

13. Procédé selon l'une des revendications 1 à 12, **caractérisé en ce que** la prothèse est une prothèse orthopédique comprenant une tige fémorale (16) et un cotyle (20) destiné à être fixé au bassin (4) et à coopérer avec la tige (16).

14. Dispositif configuré pour mettre en oeuvre un procédé de sélection d'une prothèse (16; 20) destinée à être posé sur un patient et selon l'une des revendications 1 à 13, **caractérisé en ce qu'**il comprend une interface graphique et un programme d'ordinateur comprenant des instructions de code de programme pour l'exécution des étapes du procédé lorsque ledit programme est exécuté sur un ordinateur.

15. Programme d'ordinateur comprenant des instructions de code de programme pour l'exécution des étapes du procédé selon l'une des revendications 1 à 13 lorsque ledit programme est exécuté sur un ordinateur.

## Patentansprüche

1. Verfahren zur Auswahl einer Prothese (16; 20), die dazu bestimmt ist, an einem Patienten angebracht zu werden, umfassend die folgenden Schritte:
(a) Erhalt eines Röntgenbildes (2) von der Zone des Körpers des Patienten, die dazu bestimmt ist, die Prothese (16; 20) zu erhalten, in digitaler Form, wobei das Bild die Darstellung eines strahlenundurchlässigen Referenzobjekts (12), das in der Nähe der Zone angeordnet ist, umfasst;
(b) Legen eines digitalen Layers (14), der die Prothese oder ein Element (16; 20) der Prothese darstellt, über das Röntgenbild (2) im Hinblick auf ihre Positionierung;
**dadurch gekennzeichnet, dass** in Schritt (b) der Layer (14; 22) einen Bezugspunkt (18; 24) umfasst, der dazu bestimmt ist über die Darstellung des Referenzobjekts (12) durch Verschieben des Layers gelegt zu werden, wobei die Größe des Layers variabel ist, so dass die Größe des Bezugspunktes (18; 24) jener der Darstellung des Referenzobjekts (12) entspricht, um die jeweiligen Maßstäbe des Röntgenbildes (2) und des Layers (14; 22) in Übereinstimmung zu bringen.

2. Verfahren nach Anspruch 1, **dadurch gekennzeichnet, dass** der Bezugspunkt eine Fläche oder eine kreisförmige Kontur (18; 24) ist.

3. Verfahren nach einem der Ansprüche 1 und 2, **dadurch gekennzeichnet, dass** der Bezugspunkt (18; 24) seitlich zu der Darstellung der Prothese oder ihres Elements (16; 20) angeordnet ist.

4. Verfahren nach einem der Ansprüche 1 bis 3, **dadurch gekennzeichnet, dass** die Außendimension(en) des Bezugspunktes (18; 24) mit den Dimensionen der Darstellung der Prothese oder ihres Elements (16; 20) korreliert sind.

5. Verfahren nach einem der Ansprüche 1 bis 4, **dadurch gekennzeichnet, dass** es derart vorgesehen ist, dass der Schritt (b) durch ein manuelles Eingreifen, vorzugsweise über eine Mensch-Maschine-Schnittstelle, erfolgt, und dass vorzugsweise in Schritt (b) die Verschiebung des Layers (14; 22) und seine Skalierung mit dem Röntgenbild (2) durch ein manuelles Eingreifen, vorzugsweise über eine Mensch-Maschine-Schnittstelle, erfolgt.

6. Verfahren nach einem der Ansprüche 1 bis 5, **dadurch gekennzeichnet, dass** der Schritt (b) die Auswahl der Prothese oder ihres Elements (16; 20) unter einer Vielzahl umfasst, wobei jede der Prothesen oder jedes ihrer Elemente auf einem spezifischen Layer dargestellt ist, wobei jeder dieser Layer einen Bezugspunkt entsprechend dem Bezugspunkt umfasst, der dazu bestimmt ist, über die Darstellung des Referenzobjekts gelegt zu werden, und wobei vorzugsweise die Vielzahl von Prothesen oder Prothesenelemente verschiedenen Größen eines gegebenen Typs entspricht, vorzugsweise verschiedenen Größen eines Femurschafts (16) einer orthopädischen Prothese (16; 20).

7. Verfahren nach Anspruch 6, **dadurch gekennzeichnet, dass** die Auswahl in Schritt (b) über eine Grafikschnittstelle (26) erfolgt.

8. Verfahren nach Anspruch 7, **dadurch gekennzeichnet, dass** die Grafikschnittstelle (26) eine Auslistung (30; 34) der verfügbaren Größen einer Prothese oder eines Prothesenelements, Mittel (28) zur Steuerung der Variation der Größe des ausgewählten Layers (14) und/oder Mittel zur Steuerung der Drehverschiebung (32; 36) des Layers umfasst, und vorzugsweise die Auflistung der Größen einer Prothese oder eines Prothesenelements an der Grafikschnittstelle (26) spezifische Flächen (30; 34) umfasst, die mit Hilfe eines auf der Schnittstelle beweglichen und manuell gesteuerten Zeigers auswählbar sind.

9. Verfahren nach Anspruch 8, **dadurch gekennzeichnet, dass** die Mittel zur Steuerung der Größenvariation und/oder der Drehung (28; 32; 36) einen Cursor umfasst, der durch einen manuell gesteuerten beweglichen Zeiger verschiebbar ist.

10. Verfahren nach einem der Ansprüche 1 bis 9, **dadurch gekennzeichnet, dass** die Prothese von mehreren Elementen (16; 20) gebildet ist, die dazu bestimmt sind, zusammengefügt zu werden, wobei jedes der Elemente auf einem spezifischen Layer (14; 22) dargestellt ist, wobei der Schritt (b) einem ersten dieser Elemente entspricht, wobei die Übereinstimmung der jeweiligen Maßstäbe des Röntgenbildes (2) und des Layers (14) des ersten Elements (16) automatisch die Übereinstimmung der jeweiligen Maßstäbe der Layer (22) der übrigen Elemente (20) mit dem Maßstab des Röntgenbildes (2) durchführt.

11. Verfahren nach einem der Ansprüche 1 bis 10, **dadurch gekennzeichnet, dass** die Prothese von mehreren Elementen (16; 20) gebildet ist, die dazu bestimmt sind, zusammengefügt zu werden, wobei jedes der Elemente auf einem spezifischen Layer (14; 22) dargestellt ist, wobei der Schritt (b) einem ersten (16) dieser Elemente entspricht, wobei das Verfahren den folgenden zusätzlichen Schritt umfasst:
(c) Legen eines digitalen Layers (22), das eines (20) der übrigen Elemente der Prothese darstellt, auf das Röntgenbild (2) im Hinblick auf seine Positionierung, wobei der Maßstab des Layers (22) mit dem Maßstab des Röntgenbildes (2) durch den Schritt (b) in Übereinstimmung gebracht wird, und
dass vorzugsweise der Schritt (c) für jedes Element iteriert ist.

12. Verfahren nach Anspruch 11, **dadurch gekennzeichnet, dass** mindestens eines der Elemente der Prothese (16; 20), vorzugsweise jedes der Elemente, mehrere verfügbare Größen umfasst.

13. Verfahren nach einem der Ansprüche 1 bis 12, **dadurch gekennzeichnet, dass** die Prothese eine orthopädische Prothese ist, umfassend einen Femurschaft (16) und eine Gelenkpfanne (20), die dazu bestimmt ist, am Becken (4) befestigt zu werden und mit dem Schaft (16) zusammenzuwirken.

14. Vorrichtung, die dazu vorgesehen ist, ein Verfahren zur Auswahl einer Prothese (16; 20), die dazu bestimmt ist, an einem Patienten angebracht zu werden, und die den Ansprüchen 1 bis 13 entspricht, einzusetzen, **dadurch gekennzeichnet, dass** sie eine Grafikschnittstelle und ein Computerprogramm umfasst, das Programmcodebefehle für die Ausführung der Schritte des Verfahrens umfasst, wenn das Programm auf einem Computer ausgeführt wird.

15. Computerprogramm, umfassend Programmcodebefehle für die Ausführung der Schritt des Verfahrens nach einem der Ansprüche 1 bis 13, wenn das Programm auf einem Computer ausgeführt wird.

## Claims

1. Method for selecting a prosthesis (16; 20) intended to be fitted to a patient, comprising the following steps:
(a) obtaining, in digital form, of a radiological image (2) of the area of the body of the patient intended to receive the prosthesis (16; 20), the image comprising the representation of a reference radio-opaque object (12) arranged in proximity to said area;
(b) superimposition, on the radiological image (2), of a digital overlay (14) representing the prosthesis or an element (16; 20) of said prosthesis for the positioning thereof;
**characterized in that**
in the step (b), the overlay (14; 22) comprises a register mark (18; 24) intended to be superimposed on the representation of the reference object (12) by moving the overlay, the size of said overlay being varied such that the size of the register mark (18; 24) corresponds to that of the representation of the reference object (12), so as to match the respective scales of the radiological image (2) and of the overlay (14; 22).

2. Method according to Claim 1, **characterized in that** the register mark is a surface or a circular outline (18; 24).

3. Method according to one of Claims 1 and 2, **characterized in that** the register mark (18; 24) is arranged laterally to the representation of the prosthesis or of its element (16; 20).

4. Method according to one of Claims 1 to 3, **characterized in that** the outer dimension or dimensions of the register mark (18; 24) are correlated with the dimensions of the representation of the prosthesis or of its element (16; 20).

5. Method according to one of Claims 1 to 4, **characterized in that** it is configured for the step (b) to be performed by manual intervention, preferentially via a human-machine interface, and preferentially in the step (b), the moving of the overlay (14; 22) and the scaling thereof with the radiological image (2) is performed by a manual intervention, preferentially via a human-machine interface.

6. Method according to one of Claims 1 to 5, **characterized in that** the step (b) comprises the selection of the prosthesis or of its element (16; 20) from a plurality, each of the prostheses or each of its elements being represented on a specific overlay, each of these overlays comprising a register mark corresponding to the register mark intended to be superimposed on the representation of the reference object, and preferentially the plurality of prostheses or of prosthesis elements corresponds to different sizes of a given type, preferentially different sizes of femoral rod (16) of an orthopaedic prosthesis (16; 20).

7. Method according to Claim 6, **characterized in that** the selection in the step (b) is made via a graphical interface (26).

8. Method according to Claim 7, **characterized in that** the graphical interface (26) comprises a listing (30; 34) of the sizes of prosthesis or of prosthesis element available, control means (28) for varying the size of the overlay (14) selected and/or control means for moving said overlay in rotation (32; 36), and preferentially the listing of the sizes of prosthesis or of prosthesis element on the graphical interface (26) comprises specific surfaces (30; 34) that can be selected by means of a mobile pointer on said interface and controlled manually.

9. Method according to Claim 8, **characterized in that** the control means for varying size and/or rotating (28; 32; 36) comprise a cursor that can be moved by a manually-controlled mobile pointer.

10. Method according to one of Claims 1 to 9, **characterized in that** the prosthesis is made up of several elements (16; 20) intended to be assembled together, each of said elements being represented on a specific overlay (14; 22); the step (b) corresponding to a first of these elements, the matching of the respective scales of the radiological image (2) and of the overlay (14) of the first element (16) automatically operating the matching of the respective scales of the overlays (22) of the remaining elements (20) with the scale of the radiological image (2).

11. Method according to one of Claims 1 to 10, **characterized in that** the prosthesis is made up of several elements (16; 20) intended to be assembled together, each of said elements being represented on a specific overlay (14; 22); the step (b) corresponding to a first (16) of these elements, the method comprises the following additional step:
(c) superimposition on the radiological image (2) of a digital overlay (22) representing one (20) of the remaining elements of the prosthesis, for the positioning thereof, the scale of said overlay (22) being matched with the scale of the radiological image (2) by the step (b), and
preferentially the step (c) is iterated for each element.

12. Method according to Claim 11, **characterized in that** at least one of the elements of the prosthesis (16; 20), preferentially each of these elements, comprises several available sizes.

13. Method according to one of Claims 1 to 12, **characterized in that** the prosthesis is an orthopaedic prosthesis comprising a femoral rod (16) and a cotyle (20) intended to be fixed to the pelvis (4) and to cooperate with the rod (16).

14. Device configured to implement a method for selecting a prosthesis (16; 20) intended to be fitted to a patient and according to one of Claims 1 to 13, **characterized in that** it comprises a graphical interface and a computer program comprising program code instructions for executing the steps of the method when said program is run on a computer.

15. Computer program comprising program code instructions for executing steps of the method according to one of Claims 1 to 13 when said program is run on a computer.
